# FASCICULE DE BREVET EUROPEEN

(11) **EP 0 623 353 B1**
(45) Date de publication et mention de la délivrance du brevet: **20.01.1999**
(21) Numéro de dépôt: 94400880.4
(22) Date de dépôt: 22.04.1994
(51) Int. Cl.: A61L 2/02, A23L 3/015

(54) **Dispositif de stérilisation à haute pression de produits**
Vorrichtung zum Hochdrucksterilisieren von Produkten
Apparatus for high pressure sterilization of products

(30) Priorité: 07.05.1993 FR 9305526
(43) Date de publication de la demande: 09.11.1994
(73) Titulaire: FRAMATOME, 92400 Courbevoie (FR)
(72) Inventeur: Lhenry, Bernard, F-71200 Le Creusot (FR)
(74) Mandataire: Lanceplaine, Jean-Claude

(56) Documents cités:
- EP-A- 0 480 422
- FR-A- 2 442 018

## Description

La présente invention a pour objet un dispositif de stérilisation à haute pression de produits, comme par exemple des fluides ou des éléments emballés, et destiné plus particulièrement aux laboratoires.

Il est connu pour stériliser des produits de les soumettre à une pression pouvant atteindre une valeur de 8000 bars.

Pour cela, on utilise le plus souvent une enceinte étanche de volume réduit dans laquelle sont placés les produits à stériliser qui sont soumis à une pression. Cette enceinte étanche est raccordée à une pompe destinée à gonfler l'intérieur de l'enceinte dans le but d'augmenter la pression interne.

Dans le cas d'un fluide à stériliser, ce fluide est contenu dans l'enceinte étanche et est soumis directement à la pression désirée obtenue à l'aide de la pompe.

Dans le cas d'un élément emballé à stériliser, cet élément est placé dans l'enceinte étanche et est soumis à la pression requise au moyen de ladite pompe.

Compte tenu de la pression élevée régnant à l'intérieur de l'enceinte étanche et nécessaire pour obtenir la stérilisation des produits, cette enceinte et l'ensemble du dispositif de stérilisation doivent pouvoir résister et être sûrs vis à vis du personnel.

Le dispositif de stérilisation doit donc être conçu pour présenter toutes les garanties de sécurité et également pour éviter les défaillances des pièces soumises à la pression et, même dans le cas où ces défaillances se produiraient, éviter les conséquences graves que cela entraineraient sur le personnel environnant.

L'invention a donc pour but de proposer un dispositif qui permet de stériliser des produits à très haute pression et qui présente toutes les garanties de sécurité nécessaire à ce type de dispositif.

L'invention a donc pour objet un dispositif de stérilisation à haute pression de produits, du type comprenant une enceinte étanche formée d'un fond percé d'un orifice relié à des moyens d'alimentation en fluide sous pression et d'un couvercle assemblé audit fond au moyen d'un organe de fixation et un piston coulissant monté de manière étanche dans l'enceinte et destiné à se déplacer dans ladite enceinte sous l'effet de la pression exercée par le fluide sur une surface active dudit piston coulissant, caractérisé en ce qu'il comprend une chambre de traitement haute pression ménagée dans le piston coulissant et destinée à recevoir le produit à stériliser et un piston fixe solidaire du couvercle destiné à pénétrer dans la chambre de traitement haute pression lors du déplacement du piston coulissant pour comprimer ledit produit à stériliser, l'extrémité libre du piston déterminant une surface active, le rapport entre les surfaces actives du piston coulissant et du piston fixe étant de 1 à 20.

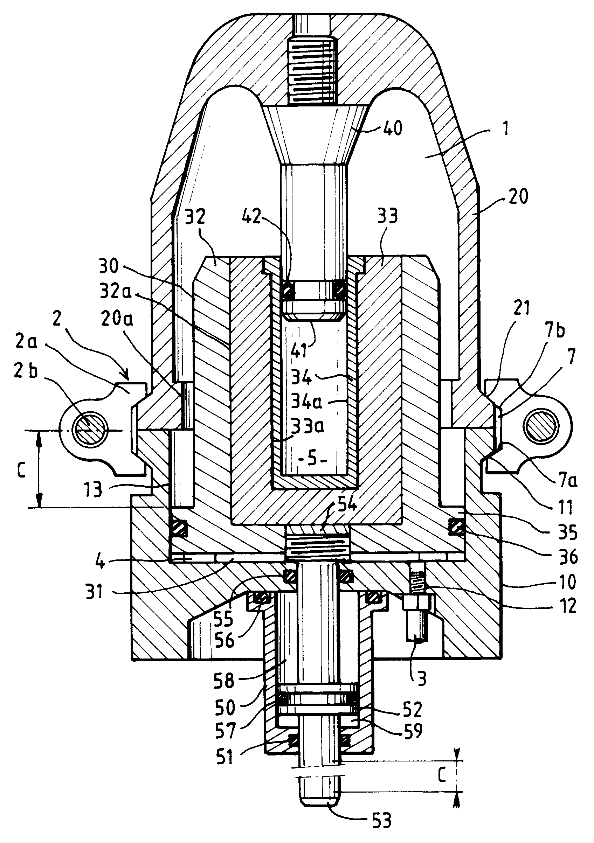

Selon d'autres caractéristiques de l'invention:
- le piston coulissant est formé d'au moins deux viroles et d'une chemise interne de protection, lesdites viroles et ladite chemise interne étant concentriques et solidaires les unes des autres,
- le piston coulissant est formé d'une virole externe, d'une virole intermédiaire et d'une chemise interne de protection, lesdites viroles et ladite chemise interne étant concentriques et solidaires les unes des autres
- la virole externe comporte un alésage borgne dans lequel est montée la virole intermédiaire,
- la virole intermédiaire est montée dans la virole externe par frettage,
- la virole intermédiaire comporte un alésage borgne concentrique à l'alésage borgne de la virole externe et dans lequel est montée la chemise centrale,
- la chemise interne comporte un alésage borgne formant la chambre de traitement et concentrique aux alésages borgnes de la virole externe et de la virole intermédiaire,
- la virole externe comporte sur sa surface extérieure un joint d'étanchéité destiné à coopérer avec la surface intérieure du fond pour assurer l'étanchéité entre, d'une part, une chambre délimitée par la surface active du piston coulissant et le fond et, d'autre part, l'enceinte,
- le piston fixe comporte sur sa surface extérieure un joint d'étanchéité destiné à coopérer avec la surface intérieure de la chemise interne pour assurer l'étanchéité entre, d'une part, la chambre de traitement et, d'autre part, l'enceinte.
   Selon une autre caractéristique de l'invention, le dispositif comprend un vérin de rappel du piston coulissant et comportant une tige traversant de manière étanche le fond, ladite tige étant reliée à la virole externe dudit piston coulissant.
   L'invention sera mieux comprise à l'aide de la description qui va suivre donnée uniquement à titre d'exemple et faite en se référant à la figure unique annexée qui représente, en coupe, un dispositif de stérilisation à haute pression, conforme à l'invention.
   Le dispositif de stérilisation représenté en coupe sur la figure comprend une enceinte étanche 1 délimitée par un fond 10 et un couvercle 20.
   Le fond 10 et le couvercle 20 sont assemblés entre eux au moyen d'un organe de fixation 2.

Cet organe de fixation 2 est constitué par un anneau de serrage 2a, soit articulé, soit formé de deux demi-anneaux solidaires entre eux par un jeu de boulons 2b.

L'anneau de serrage 2a comporte un évidement intérieur 7 en forme "V" dont un premier pan incliné 7a prend appui sur une surface inclinée 11 formée sur la surface extérieure de la partie supérieure du fond 10 et dont un second pan incliné 7b prend appui sur une surface inclinée 21 formée sur la surface extérieure de la partie inférieure du couvercle 20.

Le fond 10 est également percé d'un orifice 12 relié à des moyens d'alimentation, non représentés, en fluide sous pression par un conduit 3.

Le fond 10 délimite un alésage 13 à fond plat dans lequel est monté un piston coulissant 30.

La surface inférieure du piston 30 et la surface inférieure de l'alésage 13 du fond 10 délimite une chambre interne 4 qui est reliée aux moyens d'alimentation en fluide sous pression par le conduit 3.

Ainsi, le piston coulissant 30 est destiné à se déplacer dans l'enceinte 1 sous l'effet de la pression exercée par le fluide sous pression sur la surface inférieure du piston 30 qui constitue une surface active 31 dudit piston coulissant 30.

Le piston coulissant 30 est formé d'au moins deux viroles 32 et 33 et d'une chemise interne 34 de protection, lesdites viroles 32 et 33 et ladite chemise interne 34 étant concentriques et solidaires les unes de autres.

Dans l'exemple représenté à la figure unique, le piston coulissant 30 est formé d'une virole externe 32, d'une virole intermédiaire 33 et d'une chemise interne 34 de protection.

Pour assurer le guidage du piston coulissant 30 lors de son déplacement dans l'alésage 13 du fond 10, la virole externe 32 comporte à sa partie inférieure une collerette 35 qui forme une butée.

D'autre part, le couvercle 20 comporte au niveau de son extrémité inférieure en appui sur le fond 10, une partie en saillie interne 20a destinée à coopérer avec la collerette 35 pour limiter la course dudit piston coulissant 30.

La virole externe 32 comporte sur sa surface extérieure au niveau de la collerette 35, un joint d'étanchéité 36 destiné à coopérer avec la surface intérieure de l'alésage 13 du fond 10 pour assurer l'étanchéité entre la chambre 4 et l'enceinte 1.

La virole externe 32 comporte un alésage borgne 32a dans lequel est montée la virole intermédiaire 33 par exemple par frettage de telle manière que la virole intermédiaire 33 soit indémontable par rapport à la virole externe 32.

La virole intermédiaire 33 comporte un alésage borgne 33a concentrique à l'alésage borgne 32a de la virole externe 32 et dans lequel est montée la chemise interne 34.

La chemise interne 34 comporte un alésage borgne 34a formant une chambre de traitement 5 haute pression et concentrique aux alésages borgnes 32a et 33a de la virole externe 32 et de la virole intermédiaire 33.

L'assemblage entre la chemise interne 34 et la virole intermédiaire 33 est réalisé dans des tolérances relativement serrées et cette chemise interne 34 a pour rôle d'éviter la corrosion sous contrainte des viroles 32 et 33.

La nature du matériau constituant la chemise interne 34 est compatible avec le produit à stériliser qui est disposé dans la chambre de traitement haute pression, comme on le verra ultérieurement.

Le dispositif de stérilisation comporte également un piston fixe 40 solidaire du couvercle 20 et disposé dans l'axe du piston coulissant 30, c'est à dire dans l'axe de la chambre de traitement 5 haute pression.

Le piston fixe 40 est destiné à pénétrer dans la chambre de traitement 5 haute pression lors du déplacement du piston coulissant 30.

L'extrémité libre du piston fixe 40 détermine une surface active 41.

Le rapport entre la surface active 31 du piston coulissant 30 et la surface active 41 du piston fixe 40 est de 1 à 20.

Le piston fixe 40 comporte sur sa surface extérieure, un joint d'étanchéité 42 destiné à coopérer avec la surface intérieure de la chemise interne 34 pour assurer l'étanchéité entre la chambre de traitement 5 haute pression et l'enceinte 1.

Le dispositif de stérilisation comporte un vérin 50 de rappel du piston coulissant 30.

Ce vérin 50 comprend, de manière classique, un corps 51 fixé à l'extérieur du fond 10, un piston 52 destiné à se déplacer dans le corps 51 et une tige 53 solidaire du piston 52 et située dans l'axe du piston coulissant 30.

La tige 53 traverse de manière étanche le fond 10 et est reliée, par exemple par vissage, à la virole externe 32 du piston coulissant 30.

Un joint d'étanchéité 54 est interposé entre l'extrémité de la tige 53 et la virole intermédiaire 33 du piston coulissant 30.

Par ailleurs, des joints d'étanchéité, respectivement 55, 56 et 57 sont interposés entre la tige 53 et le fond 10, le corps 51 et le fond 10 et le piston 52 et le corps 51 du vérin 50.

Le piston 52 du vérin 50 délimite avec le corps 51 dudit vérin 50 deux chambres, respectivement 58 et 59, reliées à des moyens d'alimentation et d'aspiration, non représentés, d'un fluide sous pression permettant de ramener le piston coulissant 30 dans sa position initiale par l'intermédiaire dudit piston 52 et de la tige 53.

Dans le cas où le produit à stériliser est constitué par un fluide, ce fluide est placé dans la chambre de traitement 5 haute pression et, dans le cas où le produit à stériliser est constitué par un élément emballé, cet élément emballé est placé dans un fluide contenu dans la chambre de traitement 5 haute pression.

La tige 53 du vérin 50 peut être reliée à des moyens de repérage électrique ou mécanique de la position du piston coulissant 30 dans l'enceinte 1.

Le dispositif de stérilisation selon l'invention fonctionne de la manière suivante.

Le fluide admis dans la chambre 4 par le conduit 3 agit sur la surface active 31 du piston coulissant 30 ce qui a pour effet de déplacer ledit piston coulissant 30 sur une course C.

Cette course C est détectée par les moyens de repérage du déplacement de la tige 53 du vérin 50 qui se déplace en même temps que le piston coulissant 30.

Au cours de son déplacement, le piston coulissant 30 est guidé par le collerette 35 qui coopère avec l'alésage 13 du fond 10 et la course du piston coulissant 30 est limitée par cette collerette 35 et la partie en saillie interne 12 du couvercle 20.

Le déplacement du piston coulissant 30 dans l'enceinte 1 a pour effet de comprimer, par l'intermédiaire du piston fixe 40, le produit contenu dans la chambre de traitement 5 haute pression et de stériliser ledit produit.

La pression maximum dans la chambre de traitement 5 peut atteindre 8000 bars (55,16.10⁶ Pa) et cette pression s'exerce soit directement sur le produit à stérilisé dans le cas d'un fluide ou soit par l'intermédiaire du liquide contenu dans ladite chambre de traitement 5 dans le cas d'un élément emballé plongé dans ledit liquide.

La pression maximum de 8000 bars (55,16.10⁶ Pa) peut être atteinte grâce au rapport entre la surface active 31 du piston coulissant 30 et la surface active 41 du piston fixe 40 qui est de 1 à 20.

Le dispositif de stérilisation selon l'invention présente l'avantage d'être composé d'un minimum de pièces, limitant ainsi les interfaces qui sont soumises à la pression maximum de 8000 bars.

En effet, la chambre de traitement 5 haute pression est délimitée par un piston coulissant 30 composé d'au moins trois pièces concentriques et par un piston fixe 40 faisant office de bouchon de ladite chambre de traitement 5 haute pression.

Le troisième élément soumis à la haute pression est constitué par le joint d'étanchéité 42 monté à l'extrémité du piston fixe 40.

Le dispositif de stérilisation selon l'invention ne comporte donc que trois éléments soumis directement à la pression maximale.

La haute pression est, par conséquent, confinée dans la chambre de traitement 5.

Une défaillance éventuelle du joint d'étanchéité 42 peut entraîner des fuites qui, habituellement, sont particulièrement dangereuses, mais qui dans le dispositif selon l'invention, sont strictement confinées dans un volume interne délimité par le fond 10 et le couvercle 20.

Une fissure éventuelle du fond 10 ou du couvercle 20 n'a aucune conséquence grave, car la pression dans l'enceinte 1 est très faible par rapport à la résistance mécanique dudit fond 10 et dudit couvercle 20.

L'assemblage du fond 10 et du couvercle 20, qui est démontable, n'est pas très sollicité du fait de sa dimension qui correspond à une conception d'un vérin à 400 bars (27,6.10⁵ Pa).

Le dispositif de stérilisation selon l'invention présente donc l'avantage de ramener les conditions d'exploitation d'un dispositif à très haute pression à des nivaux de contraintes d'environnement correspondant à des dispositifs à pression conventionnelle.

Le dispositif de stérilisation selon l'invention présente donc toutes les garanties de sécurité indispensables à l'utilisation d'une très haute pression.

## Revendications

1. Dispositif de stérilisation à haute pression de produits, comprenant une enceinte étanche (1) formée d'un fond (10) percé d'un orifice (12) relié à des moyens d'alimentation en fluide sous pression et d'un couvercle (20) assemblé audit fond au moyen d'un organe de fixation (2) et un piston coulissant (30) monté de manière étanche dans l'enceinte (1) et destiné à se déplacer dans ladite enceinte (1) sous l'effet de la pression exercée par le fluide sur une surface active (31) dudit piston coulissant (30), caractérisé en ce qu'il comprend une chambre de traitement (5) haute pression ménagée dans le piston coulissant (30) et destinée à recevoir le produit à stériliser et un piston fixe (40) solidaire du couvercle (20) destiné à pénétrer dans la chambre de traitement (5) haute pression lors du déplacement du piston coulissant (30) pour comprimer ledit produit à stériliser, l'extrémité libre du piston fixe (40) déterminant une seconde surface active (41), le rapport entre les surfaces actives (31, 41) du piston coulissant (30) et du piston fixe (40) étant de 1 à 20.

2. Dispositif selon la revendication 1, caractérisé en ce que le piston coulissant (30) est formé d'au moins deux viroles (32, 33) et d'une chemise interne (34) de protection, lesdites viroles (32, 33) et ladite chemise interne (34) étant concentriques et solidaires les unes des autres.

3. Dispositif selon la revendication 2, caractérisé en ce que le piston coulissant (30) est formé d'une virole externe (32), d'une virole intermédiaire et d'une chemise interne (34) de protection, lesdites viroles (32, 33) et ladite chemise interne (34) étant concentriques et solidaires les unes des autres.

4. Dispositif selon l'une des revendications 1 à 3, caractérisé en ce que la virole externe (32) comporte un alésage borgne (32a) dans lequel est montée la virole intermédiaire (33).

5. Dispositif selon la revendication 4, caractérisé en ce que la virole intermédiaire (33) est montée dans la virole externe par frettage.

6. Dispositif selon l'une des revendications 1 à 4, caractérisé en ce que la virole intermédiaire (33) comporte un alésage borgne (33a) concentrique à l'alésage borgne (32a) de la virole externe (32) et dans lequel est montée la chemise interne (34).

7. Dispositif selon l'une des revendications précédentes, caractérisé en ce que la chemise interne (34) comporte un alésage borgne (34a) formant la chambre de traitement (5) haute pression et concentrique aux alésages borgnes (32a, 33a) de la virole externe (32) et de la virole intermédiaire (33).

8. Dispositif selon l'une des revendications précédentes, caractérisé en ce que la virole externe (32) comporte sur sa surface extérieure un joint d'étanchéité (36) destiné à coopérer avec la surface intérieure du fond (10) pour assurer l'étanchéité entre, d'une part, une chambre (4) délimitée par la surface active (31) du piston coulissant (30) et le fond (10) et, d'autre part, l'enceinte (1).

9. Dispositif selon l'une des revendication 1 à 7, caractérisé en ce que le piston fixe (40) comporte sur sa surface extérieure un joint d'étanchéité (42) destiné à coopérer avec la surface intérieure de la chemise interne (34) pour assurer l'étanchéité entre, d'une part, la chambre de traitement (5) haute pression et, d'autre part, l'enceinte (1).

10. Dispositif selon la revendication 1, caractérisé en ce qu'il comporte un vérin (50) de rappel du piston coulissant (30).

11. Dispositif selon la revendication 10, caractérisé en ce que le vérin (50) comporte une tige (53) traversant de manière étanche le fond (10) et reliée à la virole externe (32) du piston coulissant (30).

12. Dispositif selon les revendications 10 et 11, caractérisé en ce que la tige (53) du vérin (50) est reliée à des moyens de repérage de la position du piston coulissant (30) dans l'enceinte (1).

## Claims

1. Device for the high-pressure sterilisation of products, comprising a fluid-tight enclosure (1) formed from a base (10) pierced with an orifice (12) connected to means of feeding with pressurised fluid and a cover (20) assembled with the said base by means of an attachment device (2) and a sliding piston (30) fitted in a fluid-tight manner in the enclosure (1) and intended to move inside the said enclosure (1) under the effect of the pressure applied by the fluid on an active surface (31) of the said sliding piston (30), characterised in that it comprises a high-pressure processing chamber (5) formed in the sliding piston (30) and intended to receive the product to be sterilised and a fixed piston (40) integral with the cover (20) intended to penetrate into the high pressure processing chamber (5) during the displacement of the sliding piston (30) in order to compress the said product to be sterilised, the free end of the fixed piston (40) determining a second active surface (41), the ratio between the active surfaces (31, 41) of the sliding piston (30) and of the fixed piston (40) being 1 to 20.

2. Device according to Claim 1, characterised in that the sliding piston (30) is formed from at least two shells (32, 33) and an inner protective liner (34), the said shells (32, 33) and the said inner liner (34) being concentric and integral with one another.

3. Device according to Claim 2, characterised in that the sliding piston (30) is formed from an outer shell (32), an intermediate shell and in inner protective liner (34), the said shells (32, 33) and the said inner liner (34) being concentric and integral with one another.

4. Device according to one of Claims 1 to 3, characterised in that the outer shell (32) comprises a blind bore (32a) inside of which the intermediate shell (33) is fitted.

5. Device according to Claim 4, characterised in that the intermediate shell (33) is fitted inside the outer shell by shrink-fitting.

6. Device according to one of Claims 1 to 4, characterised in that the intermediate shell (33) comprises a blind bore (33a) which is concentric with the blind bore (32a) of the outer shell (32) and inside of which the inner liner (34) is fitted.

7. Device according to one of the preceding claims, characterised in that the inner liner (34) comprises a blind bore (34a) forming the high-pressure processing chamber (5) and which is concentric with the blind bores (32a, 33a) of the outer shell (32) and of the intermediate shell (33).

8. Device according to one of the preceding claims, characterised in that the outer shell (32) comprises on its outer surface a seal (36) intended to co-operate with the inside surface of the base (10) in order to ensure fluid-tightness between, on the one hand, a chamber (4) delimited by the active surface (31) of the sliding piston (30) and the base (10) and, on the other hand, the enclosure (1).

9. Device according to one of Claims 1 to 7, characterised in that the fixed piston (40) comprises on its outer surface a seal (42) intended to co-operate with the inside surface of the inner liner (34) in order to ensure fluid-tightness between, on the one hand, the high-pressure processing chamber (5) and, on the other hand, the enclosure (1).

10. Device according to Claim 1, characterised in that it comprises a jack (50) for returning the sliding piston (30).

11. Device according to Claim 10, characterised in that the jack (50) comprises a rod (53) traversing the base (10) in a fluid-tight manner and connected to the outer shell (32) of the sliding piston (30).

12. Device according to Claim 10 and 11, characterised in that the rod (53) of the jack (50) is connected to means of marking the position of the sliding piston (30) in the enclosure (1).

## Patentansprüche

1. Vorrichtung zum Sterilisieren bei hohem Druck von Produkten, mit einer dichten Hülle (1), die aus einem Boden (10), der mit einer Öffnung (12) durchsetzt ist, die mit Zuführeinrichtungen von Druckflüssigkeit verbunden ist, und aus einem Deckel (20) gebildet ist, der mit dem Boden mittels eines Befestigungsorgans (2) und einem Gleitkolben (30) zusammengesetzt ist, der auf dichtende Weise in der Hülle (1) angebracht ist und dazu dient, sich in dieser Hülle (1) unter der Einwirkung des Druckes zu bewegen, der von dem Fluid auf eine aktive Oberfläche (31) des Gleitkolbens (30) ausgeübt wird, dadurch gekennzeichnet, daß sie eine Hochdruckbehandlungskammer (5) aufweist, die in dem Gleitkolben (30) ausgebildet ist, und dazu dient, das zu sterilisierende Produkt aufzunehmen, und einen festen Kolben (40) aufweist, der mit dem Deckel (20) verbunden ist und dazu dient, in die Hochdruck-Behandlungskammer (5) bei der Bewegung des Gleitkolbens (30) einzudringen, um das zu sterilisierende Produkt zu komprimieren, wobei das freie Ende des festen Kolbens (5) eine zweite aktive Oberfläche (41) bildet, und wobei das Verhältnis zwischen den aktiven Oberflächen (31, 41) des Gleitkolbens (30) und des festen Kolbens (40) zwischen 1 und 20 beträgt.

2. Vorrichtung nach Anspruch 1, dadurch gekennzeichnet, daß der Gleitkolben (30) aus wenigstens zwei Ringen (32, 33) und einer inneren Schutzschürze (34) gebildet ist, wobei die Ringe (32, 33) und die innere Schürze (34) konzentrisch angeordnet und miteinander verbunden sind.

3. Vorrichtung nach Anspruch 2, dadurch gekennzeichnet, daß der Gleitkolben (30) aus einem äußeren Ring (32), einem Zwischenring und einer inneren Schutzschürze (34) gebildet ist, wobei die Ringe (32, 33) und die innere Schürze (34) konzentrisch angeordnet und miteinander verbunden sind.

4. Vorrichtung nach einem der Ansprüche 1 bis 3, dadurch gekennzeichnet, daß der äußere Ring (32) ein Sackloch (32a) aufweist, in dem der Zwischenring (33) montiert ist.

5. Vorrichtung nach Anspruch 4, dadurch gekennzeichnet, daß der Zwischenring (33) in den äußeren Ring durch Bandagierung montiert ist.

6. Vorrichtung nach einem der Ansprüche 1 bis 4, dadurch gekennzeichnet, daß der Zwischenring (33) ein Sackloch (33a) aufweist, das konzentrisch zum Sackloch (32a) des äußeren Rings (32) ist und in dem die innere Schürze (34) angebracht ist.

7. Vorrichtung nach einem der vorhergehenden Ansprüche, dadurch gekennzeichnet, daß die innere Schürze (34) ein Sackloch (34a) aufweist, das die Hochdruck-Behandlungskammer (5) bildet und konzentrisch zu den Sacklöchern (32a, 33a) des äußeren Rings (32) und des Zwischenrings (33) ist.

8. Vorrichtung nach einem der vorhergehenden Ansprüche, dadurch gekennzeichnet, daß dar äußere Ring (32) an seiner Außenseite eine Dichtung (36) aufweist, die dazu dient, mit der Innenseite des Bodens (10) zusammenzuwirken, um die Dichtheit zwischen einerseits einer Kammer (4), die von der aktiven Oberfläche (31) des Gleitkolbens (30) begrenzt ist, und dem Boden (10) und, andererseits, der Hülle (1) sicherzustellen.

9. Vorrichtung nach einem der Ansprüche 1 bis 7, dadurch gekennzeichnet, daß der feste Kolben (40) an seiner Außenseite eine Dichtung (42) aufweist, die dazu dient, mit der Innenseite der inneren Schürze (34) zusammenzuwirken, um die Dichtheit zwischen, einerseits der Hochdruckbehandlungskammer (5) und andererseits der Hülle (1) sicherzustellen.

10. Vorrichtung nach Anspruch 1, dadurch gekennzeichnet, daß sie einen Rückdruckstelltrieb (50) des Gleitkolbens (30) aufweist.

11. Vorrichtung nach Anspruch 10, dadurch gekennzeichnet, daß der Stelltrieb (50) eine Stange (53) aufweist, die auf dichtende Weise den Boden (10) durchsetzt und mit dem äußeren Ring (32) des Gleitkolbens (30) verbunden ist.

12. Vorrichtung nach Anspruch 10 und 11, dadurch gekennzeichnet, daß die Stange (53) des Stelltriebs (50) mit Positionsauffangeinrichtungen des Gleitkolbens (30) in der Hülle (1) verbunden ist.
